# EUROPEAN PATENT APPLICATION

(11) **EP 3 984 618 A1**
(43) Date of publication of application: **20.04.2022**
(21) Application number: 20851402.6
(22) Date of filing: 03.09.2020
(51) Int. Cl.: B01D 39/14, B01D 39/16, A61L 9/01, A61L 9/16, B01D 39/20, B01D 46/00, B01D 46/54, B01J 20/28, B01J 20/32, F24F 3/16

(54) **FILTER MEDIUM FOR AIR PURIFICATION AND METHOD FOR PRODUCING SAME**

(71) Applicant: Nikki-Universal Co., Ltd., Shinagawa-ku Tokyo 141-8563 (JP); Hokuetsu Corporation, Nagaoka-shi Niigata 940-0027 (JP)
(72) Inventor: The designation of the inventor has not yet been filed
(74) Representative: McWilliams, David John
(86) International application number: PCT/JP2020/033438
(87) International publication number: WO 2022/049696

(57) **Abstract**

The present disclosure relates to an air purification filter including a substrate, and an enzyme and glycine supported on the substrate, wherein the enzyme includes at least one enzyme selected from the group consisting of β-1,3-glucanase, chitinase, lysozyme, protease, glycosidase, β-galactosidase, endo-β-N-acetylglucosaminidase and endolysine.

## Description

### Technical Field

The present invention relates to an air purification filter and a method of producing the same.

### Background Art

An air purification filter on which an enzyme and a modified enzyme modified with a specific compound are supported is known (for example, Patent Literature 1).

### Citation List

### Patent Literature

[Patent Literature 1] PCT International Publication No. WO 2004/035173

### Summary of Invention

### Technical Problem

Although the air purification filter described in Patent Literature 1 has an excellent bactericidal property, further improvement in properties is required currently.

An object of the present invention is to provide an air purification filter having an excellent bactericidal property. Another object of the present invention is to provide a method of producing such an air purification filter.

### Solution to Problem

An air purification filter according to one aspect of the present invention includes a substrate, and an enzyme and glycine supported on the substrate. Here, the enzyme includes at least one enzyme selected from the group consisting of β-1,3-glucanase, chitinase, lysozyme, protease, glycosidase, β-galactosidase, endo-β-N-acetylglucosaminidase and endolysine.

Such an air purification filter has a better bactericidal property than a conventional filter. The reason for this is not clear, but it is speculated that glycine enhances properties of an enzyme (lytic enzyme).

In one aspect, the enzyme may be modified with at least one modifying agent selected from the group consisting of N-substituted carbamate bromide, N-substituted imide carbonate bromide, dimethylaminoethyl, diethylaminoethyl, protamine, polyethyleneimine, polyvinylamine, polyallylamine, polylysine, polyornithine, dextran, dextran sulfate, dextrin and chondroitin sulfate.

In one aspect, the substrate may be composed of at least one selected from the group consisting of inorganic fibers, natural fibers, recycled fibers and organic synthetic fibers.

In one aspect, the ratio of a supported amount of the glycine to a supported amount of the enzyme (supported amount of glycine/supported amount of enzyme) may be 0.1 to 100.

In one aspect, the supported amount of the enzyme may be 0.01 to 4.0 mass% based on a total amount of the air purification filter.

A method of producing an air purification filter according to one aspect of the present invention includes a papermaking process of obtaining wet paper or dry paper using substrate fibers; a contact process of bringing a treatment solution containing an enzyme and glycine into contact with the wet paper or dry paper, and a drying process of removing volatile components from the wet paper or the dry paper that has come into contact with the treatment solution. Here, the enzyme includes at least one selected from the group consisting of β-1,3-glucanase, chitinase, lysozyme, protease, glycosidase, β-galactosidase, endo-β-N-acetylglucosaminidase and endolysine. The air purification filter obtained by this production method has a better bactericidal property than a filter obtained by a conventional method.

In one aspect, the treatment solution may further include at least one modifying agent selected from the group consisting of N-substituted carbamate bromide, N-substituted imide carbonate bromide, dimethylaminoethyl, diethylaminoethyl, protamine, polyethyleneimine, polyvinylamine, polyallylamine, polylysine, polyornithine, dextran, dextran sulfate, dextrin and chondroitin sulfate.

### Advantageous Effects of Invention

According to the present invention, it is possible to provide an air purification filter having an excellent bactericidal property. In addition, according to the present invention, it is possible to provide a method of producing such an air purification filter.

### Brief Description of Drawings

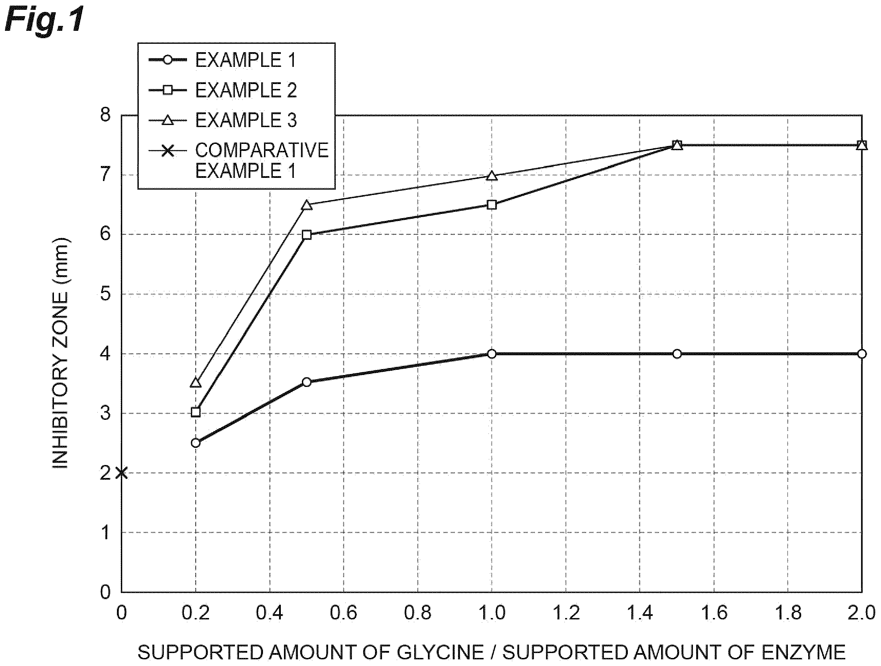

FIG. 1 is a diagram showing antibacterial performance of filters obtained in examples.

### Description of Embodiments

<Air purification filter>

An air purification filter includes a substrate, and an enzyme and glycine supported on the substrate. The filter may contain a resin binder and/or a solid binder for binding substrate fibers.

### (Substrate)

The substrate is composed of at least one selected from the group consisting of inorganic fibers, natural fibers, recycled fibers and organic synthetic fibers. The thickness of the substrate (the thickness of the air purification filter) can be, for example, 0.1 to 0.6 mm.

The material of the substrate fibers (fibers constituting the substrate) is not particularly limited as long as it can function as a filter. However, the substrate fiber preferably has a functional group that can interact with or bind to an enzyme, for example, a hydroxy group, a carboxylic group, an amino group, an amide group, an imino group or the like on its surface. Examples of substrate fibers include inorganic fibers such as borosilicate glass fibers and silica alumina fibers, natural fibers such as wood pulp and cotton linters, recycled fibers such as rayon fibers and lyocell fibers, and organic synthetic fibers such as vinylon fibers and polyamide fibers.

Here, in order to support an enzyme favorably, it is preferable that ionic polarity of the entire substrate fibers constituting the filter and the ionic polarity of the enzyme having a bactericidal property be opposite to each other. Therefore, the substrate fiber preferably has at least one functional group among a hydroxy group and a carboxylic group which have anionic polarity, and an amino group, an amide group and an imino group which have cationic polarity. When the support of the enzyme is favorable, since the surface of the substrate fiber is uniformly and efficiently covered with an enzyme, a bactericidal property of the filter is easily improved and an adverse effect on the filtration performance is easily reduced.

### (Enzyme)

Examples of enzymes include β-1,3-glucanase, chitinase, lysozyme, protease, glycosidase, β-galactosidase, endo-β-N-acetylglucosaminidase and endolysine.

The enzyme may be modified with at least one modifying agent selected from the group consisting of N-substituted carbamate bromide, N-substituted imide carbonate bromide, dimethylaminoethyl, diethylaminoethyl, protamine, polyethyleneimine, polyvinylamine, polyallylamine, polylysine, polyornithine, dextran, dextran sulfate, dextrin and chondroitin sulfate. These compounds have common properties in that they are proteins and peptides having antibacterial activity. An enzyme modified with a modifying agent can be called a modified enzyme. On the substrate, all the enzymes used as a raw material may be modified with a modifying agent or some of the enzymes may be modified. That is, the substrate may support at least one of an (unmodified) enzyme and a modified enzyme or may support both of them.

The supported amount of the enzyme can be 0.01 to 4.0 mass% or may be 0.05 to 4.0 mass% based on a total amount of the air purification filter. When the supported amount is 0.01 mass% or more, a function of the enzyme is easily sufficiently expressed, and a practical effect is easily obtained. On the other hand, since the amount supported on the substrate is limited, the upper limit of the supported amount can be about 4.0 mass%. Even if the supported amount is more than this, it is difficult to immobilize the enzyme on the substrate fibers. Here, the supported amount of the enzyme indicates the supported amount of the enzyme itself, and it does not include the amount of the modifying agent in the case of the modified enzyme.

The ratio of the supported amount of glycine to the supported amount of the enzyme (supported amount of glycine/supported amount of enzyme) can be 0.1 to 100 or may be 0.1 to 50, 0.1 to 10 or 0.2 to 2. When the ratio is 0.1 or more, antibacterial performance tends to be easily improved according to an interaction between the enzyme and glycine. On the other hand, when the ratio is 100 or less, the filter strength tends to be easily maintained.

### (Resin binder)

The resin binder is provided in the form of an aqueous solution, an aqueous dispersion or an aqueous suspension, and mixed into a treatment solution containing an enzyme or the like, and is applied to a substrate in the contact process. The strength of the filter is improved by binding substrate fibers constituting the filter with the resin binder. Here, in consideration of non-reactivity with the enzyme, the ionic polarity of the entire substrate fibers and the ionic polarity of the resin binder may be opposite to each other. That is, it is preferable that the enzyme and the resin binder have the same ionic polarity.

Examples of resin binders include cationic synthetic resin binders, anionic synthetic resin binders and nonionic synthetic resin binders. Cationic synthetic resin binders have weak cations to strong cations, and anionic synthetic resin binders have weak anions to strong anions.

Examples of cationic synthetic resin binders include acrylic resins, for example, Salvinol AD-7 (manufacturer: Saiden Chemical Industry Co., Ltd.), urethane resins, for example, Superflex 600 (manufacturer: DKS Co., Ltd.), vinyl acetate resins, for example, Movinyl 350 (manufacturer: Japan Coating Resin Co., Ltd.,) and SBR resins, for example, Cementex C-360 (manufacturer: Obanaya Cementex Co., Ltd.). At least one of these cationic synthetic resin binders can be used.

Examples of anionic synthetic resin binders include acrylic resins, for example, Bonkote AN155 (manufacturer: Dainippon Ink and Chemicals, Inc.), urethane resins, for example, Superflex 700 (manufacturer: DKS Co., Ltd.), vinyl acetate resins, for example, Polysol AX-751 (manufacturer: Showa Denko K.K.), and SBR resins, for example, Luckstar 7300A (manufacturer: Dainippon Ink and Chemicals, Inc.). At least one of these anionic synthetic resin binders can be used.

Examples of nonionic synthetic resin binders include acrylic resins, for example, AE887 (manufacturer: E-TEC), urethane resins, for example, Superflex 500M (manufacturer: DKS Co., Ltd.), vinyl acetate resins, for example, Movinyl 50M (manufacturer: Japan Coating Resin Co., Ltd.), and SBR resins, for example, Nalstar SR-100 (manufacturer: Nippon A&L Inc.). At least one of these nonionic synthetic resin binders can be used.

The amount of the resin binder can be 0.1 to 10.0 mass% or may be 0.5 to 7.0 mass% based on a total amount of the air purification filter. When the amount is 0.1 mass% or more, a sufficient binding action of the substrate fibers tends to be obtained. On the other hand, when the amount is 10.0 mass% or less, a sufficient bactericidal property with an enzyme tends to be maintained.

By adjusting the ionic polarities of the enzyme and the resin binder with respect to the substrate fibers constituting the filter as described above, the normal tensile strength, the wet tensile strength indicating water resistance, and water repellency of the filter are further improved.

For example, when substrate fibers to be used have a hydroxy group or a carboxylic group (have anionic polarity), it is preferable to use a cationic synthetic resin binder. In addition, when substrate fibers to be used have an amino group, an amide group or an imino group (have cationic polarity), it is preferable to use an anionic synthetic resin binder. When a mixture of substrate fibers having anionic polarity and substrate fibers having cationic polarity is used, the enzyme and the resin binder may be selected according to the mixing ratio.

### (Solid binder)

In order to improve the strength of the filter, a solid binder can be used. The solid binder is in the form of powder or fiber, and is disaggregated and subjected to papermaking together with the substrate fibers, all or some of the components in the drying process after the contact process are melted, and the substrate fibers are adhered to each other. Regarding the solid binder, vinylon fibers, polyester fibers, polyolefin fibers, and the like can be used. A solid binder can be used alone without using the resin binder or may be used together with the resin binder.

The amount of the solid binder can be 0.1 to 95.0 mass% and may be 0.5 to 80.0 mass% based on a total amount of the air purification filter. Alternatively, the amount of the solid binder can be 0.1 to 1,900 mass% and may be 0.5 to 400 mass% with respect to the mass of the substrate fibers. When the amount is the lower limit value or more, the binding action of the substrate fibers can be easily obtained. On the other hand, when the amount is the upper limit value or less, the blending amount of the substrate fibers required for filtration can be secured.

As other components, a water-repellent component composed of a fluorine resin, a silicone resin, a paraffin wax, an alkyl ketene dimer, an alkenyl succinic anhydride or the like may be supported on the substrate.

### <Method of producing air purification filter>

A method of producing an air purification filter includes a papermaking process of obtaining wet paper or dry paper using substrate fibers; a contact process of bringing a treatment solution containing an enzyme and glycine into contact with the wet paper or dry paper, and a drying process of removing volatile components from the wet paper or the dry paper that has come into contact with the treatment solution. The treatment solution may further include at least one modifying agent selected from the group consisting of N-substituted carbamate bromide, N-substituted imide carbonate bromide, dimethylaminoethyl, diethylaminoethyl, protamine, polyethyleneimine, polyvinylamine, polyallylamine, polylysine, polyornithine, dextran, dextran sulfate, dextrin and chondroitin sulfate. When the modifying agent is used, the ratio of the amount of the modifying agent to the amount of the enzyme (amount of modifying agent/amount of enzyme) can be 0.2 to 4 and may be 0.5 to 2. When the ratio is 0.2 or more, a modifying effect of the enzyme with a modifying agent tends to be easily obtained. On the other hand, when the ratio is 4 or less, excellent antibacterial performance tends to be easily obtained.

### (Papermaking process)

The optimal type and fiber diameter of the substrate fiber are selected in consideration of physical properties such as a pressure loss, collection performance, and a basis mass of a desired filter. For example, in the case of an HEPA filter, as an example, substrate fibers containing 95% of ultra-fine glass fibers having an average fiber diameter of 3 µm or less and 5% of chopped strand glass fibers are prepared. In addition, in the case of a medium-performance filter, as an example, substrate fibers containing 50% of ultra-fine glass fibers having an average fiber diameter of 3 µm or less and 50% of chopped strand glass fibers are prepared. A slurry containing the prepared substrate fibers and a solid binder as necessary is prepared and then dehydrated using a wet papermaking machine or the like to obtain wet paper or dry paper obtained by drying wet paper. Here, the wet paper has a water content of 10 to 90 mass%, preferably 20 to 80 mass%, and the dry paper has a water content of less than 10 mass%.

### (Contact process)

An enzyme and glycine, and as necessary, a modifying agent, a resin binder and the like are mixed together with a liquid component to obtain a treatment solution (in a solution state or a suspension state). The liquid component may be an aqueous component, a non-aqueous component such as an alcohol, acetone, or hexane, or a mixed component thereof. However, in consideration of dispersibility of the components, an aqueous component is preferable. The amount of each component added to the liquid component may be appropriately adjusted so that the supported amount on the substrate is a desired amount. The enzyme may be modified with a modifying agent in advance. That is, a modified enzyme may be used. Examples of a method of bringing wet paper or dry paper into contact with a treatment solution include a method of impregnating the wet paper or dry paper with a treatment solution, a method of spraying a treatment solution to the wet paper or dry paper, and a roll transfer method.

### (Drying process)

The drying process can be performed using a dryer alone such as a cylinder dryer, a Yankee dryer, an air-through dryer, a rotary dryer, or an infrared dryer, or a combination thereof. The drying temperature can be 80 °C to 220 °C and may be 100 °C to 200 °C. The drying time may be appropriately adjusted according to the composition of the filter.

The air purification filter obtained in this manner can be used in various situations for commercial use and general household use in which an air filter is required. In particular, it is most suitable for commercial use in food factories, drinking water factories, pharmaceutical factories, animal experiment facilities, hospital facilities, semiconductor facilities, and bio facilities.

### [Examples]

The present invention will be described below in more detail with reference to examples. However, the present invention is not limited to these examples.

### (Example 1)

95 mass% of ultra-fine glass fibers having a hydroxy group as a functional group having anionic polarity and having an average fiber diameter of 3 µm or less and 5 mass% of chopped strand glass fibers having an average fiber diameter of 9 µm were disaggregated with a pulper at a concentration of 0.4 mass% using acid water having a pH of 3.5 to prepare a slurry. Wet paper was dehydrated from the slurry using a wet papermaking machine. In addition, 3 mass% of lysozyme as an enzyme, 0.6, 1.5, 3.0, 4.5, or 6.0 mass% of glycine, 3 mass% of a cationic synthetic resin binder (Salvinol AD-7, commercially available from Saiden Chemical Industry Co., Ltd.), and 93.4, 92.5, 91.0, 89.5, or 88.0 mass% of water were mixed to prepare a mixed solution in an aqueous solution state. The wet paper obtained above was impregnated with the mixed solution so that 3 mass% of the solid content in the mixed solution was supported with respect to the mass of the dried filter. Then, dehydration was performed and drying was performed in a rotary dryer at 120 °C to obtain an HEPA filter having a thickness of 0.30 mm and a basis mass of 65 g/m².

### (Example 2)

Wet paper was obtained in the same manner as in Example 1. In addition, 3 mass% of lysozyme as an enzyme, 3 mass% of protamine as a modifying agent, 0.6, 1.5, 3.0, 4.5, or 6.0 mass% of glycine, 3 mass% of a cationic synthetic resin binder (Salvinol AD-7, commercially available from Saiden Chemical Industry Co., Ltd.) and 90.4, 89.5, 88.0, 86.5, or 85.0 mass% of water were mixed to prepare a mixed solution in an aqueous solution state. The wet paper obtained above was impregnated with the mixed solution so that 3 mass% of the solid content in the mixed solution was supported with respect to the mass of the dried filter. Then, dehydration was performed and drying was performed in a rotary dryer at 120 °C to obtain an HEPA filter having a thickness of 0.30 mm and a basis mass of 65 g/m².

### (Example 3)

95 mass% of ultra-fine glass fibers having a hydroxy group as a functional group having anionic polarity and having an average fiber diameter of 3 µm or less and 5 mass% of chopped strand glass fibers having an average fiber diameter of 9 µm and 4 mass% of the solid binder (vinylon SPG056-11 commercially available from Kuraray Co., Ltd.) with respect to the mass of these substrate fibers were disaggregated with a pulper at a concentration of 0.4 mass% using acid water having a pH of 3.5 to prepare a slurry. Wet paper was dehydrated from the slurry using a wet papermaking machine. In addition, 3 mass% of lysozyme as an enzyme, 3 mass% of protamine as a modifying agent, 0.6, 1.5, 3.0, 4.5, or 6.0 mass% of glycine and 93.4, 92.5, 91.0, 89.5, or 88.0 mass% of water were mixed to prepare a mixed solution in an aqueous solution state. The wet paper obtained above was impregnated with the mixed solution so that 3 mass% of the solid content in the mixed solution was supported with respect to the mass of the dried filter. Then, dehydration was performed and drying was performed in a rotary dryer at 120 °C to obtain an HEPA filter having a thickness of 0.30 mm and a basis mass of 65 g/m².

### (Comparative Example 1)

Wet paper was obtained in the same manner as in Example 1. In addition, 3 mass% of lysozyme as an enzyme, 3 mass% of protamine as a modifying agent, 3 mass% of a cationic synthetic resin binder (Salvinol AD-7, commercially available from Saiden Chemical Industry Co., Ltd.) and 91 mass% of water were mixed to prepare a mixed solution in an aqueous solution state. The wet paper obtained above was impregnated with the mixed solution so that 3 mass% of the solid content in the mixed solution was supported with respect to the mass of the dried filter. Then, dehydration was performed and drying was performed in a rotary dryer at 120 °C to obtain an HEPA filter having a thickness of 0.30 mm and a basis mass of 65 g/m².

### (Comparative Example 2)

Wet paper was obtained in the same manner as in Example 1. In addition, 3 mass% of protamine, 0.6, 1.5, 3.0, 4.5, or 6.0 mass% of glycine, 3 mass% of a cationic synthetic resin binder (Salvinol AD-7, commercially available from Saiden Chemical Industry Co., Ltd.) and 93.4, 92.5, 91.0, 89.5, or 88.0 mass% of water were mixed to prepare a mixed solution in an aqueous solution state. The wet paper obtained above was impregnated with the mixed solution so that 3 mass% of the solid content in the mixed solution was supported with respect to the mass of the dried filter. Then, dehydration was performed and drying was performed in a rotary dryer at 120 °C to obtain an HEPA filter having a thickness of 0.30 mm and a basis mass of 65 g/m².

### (Evaluation)

A test of evaluating an antibacterial property based on the size of a halo (bacterial growth inhibitory zone) formed around an antibacterial test piece was performed. A sample (3×3×3.14 mm²) having a diameter of 6 mm was randomly cut from the filter obtained in each example and used as a test piece. *Micrococcus luteus* was sprayed to the test piece and the sprayed bacteria inhibitory zone was measured. The outline of the test method is shown below.
(1) A liquid aqueous solution (concentration: 1×10⁷ CFU/filter) prepared by culturing in a heart infusion liquid medium and performing centrifuging and washing was dispersed and added dropwise to all the required number of test pieces to be evaluated.
(2) The above test pieces were naturally dried in a biosafety cabinet for a specified time and bacteria were then extracted with a vibration mixer using a phosphate buffer solution.
(3) The extracted undiluted solution and diluted solution were transplanted in a standard agar medium.
(4) After culturing at 35 °C for 24 hours, the bacterial growth inhibitory zone was measured with a ruler.

FIG. 1 is a diagram showing antibacterial performance of filters obtained in examples. Specifically, FIG. 1 shows the antibacterial performance with respect to the amount of glycine added when the amount of the enzyme was fixed. As shown in FIG. 1, the filters of the Examples had an excellent antibacterial property. Here, in Comparative Example 2, the inhibitory zone was 1 mm.

## Claims

1. An air purification filter, comprising:
a substrate, and an enzyme and glycine supported on the substrate,
wherein the enzyme includes at least one enzyme selected from the group consisting of β-1,3-glucanase, chitinase, lysozyme, protease, glycosidase, β-galactosidase, endo-β-N-acetylglucosaminidase and endolysine.

2. The air purification filter according to claim 1, wherein the enzyme is modified with at least one modifying agent selected from the group consisting of N-substituted carbamate bromide, N-substituted imide carbonate bromide, dimethylaminoethyl, diethylaminoethyl, protamine, polyethyleneimine, polyvinylamine, polyallylamine, polylysine, polyornithine, dextran, dextran sulfate, dextrin and chondroitin sulfate.

3. The air purification filter according to claim 1 or 2, wherein the substrate is composed of at least one selected from the group consisting of inorganic fibers, natural fibers, recycled fibers and organic synthetic fibers.

4. The air purification filter according to any one of claims 1 to 3, wherein the ratio of a supported amount of the glycine to a supported amount of the enzyme (supported amount of glycine/supported amount of enzyme) is 0.1 to 100.

5. The air purification filter according to any one of claims 1 to 4, wherein the supported amount of the enzyme is 0.01 to 4.0 mass% based on a total amount of the air purification filter.

6. A method of producing an air purification filter, comprising:
a papermaking process of obtaining wet paper or dry paper using substrate fibers;
a contact process of bringing a treatment solution containing an enzyme and glycine into contact with the wet paper or the dry paper; and
a drying process of removing volatile components from the wet paper or the dry paper that has come into contact with the treatment solution,
wherein the enzyme includes at least one selected from the group consisting of β-1,3-glucanase, chitinase, lysozyme, protease, glycosidase, β-galactosidase, endo-β-N-acetylglucosaminidase and endolysine.

7. The production method according to claim 6, wherein the treatment solution further includes at least one modifying agent selected from the group consisting of N-substituted carbamate bromide, N-substituted imide carbonate bromide, dimethylaminoethyl, diethylaminoethyl, protamine, polyethyleneimine, polyvinylamine, polyallylamine, polylysine, polyornithine, dextran, dextran sulfate, dextrin and chondroitin sulfate.
